# EUROPEAN PATENT APPLICATION

(11) **EP 4 681 723 A1**
(43) Date of publication of application: **21.01.2026**
(21) Application number: 24769664.4
(22) Date of filing: 24.01.2024
(51) Int. Cl.: A61K 38/08, A61K 38/17, A61K 48/00, G01N 33/574, A61P 35/00

(54) **PHARMACEUTICAL COMPOSITION FOR TREATING TRIPLE-NEGATIVE BREAST CANCER**

(30) Priority: 15.03.2023 US 202363452273 P
(71) Applicant: Hou, Ming-Hung, Taichung, Taiwan 402 (TW)
(72) Inventor: Hou, Ming-Hung, Taichung, Taiwan 402 (TW)
(74) Representative: Lang, Christian
(86) International application number: PCT/CN2024/073807
(87) International publication number: WO 2024/187968

(57) **Abstract**

A pharmaceutical composition for treating triple-negative breast cancer is provided. The active ingredients of the pharmaceutical composition include actinomycin D and doxorubicin, both of which are chemotherapy drugs. The synergistic effect between actinomycin D and doxorubicin enables the pharmaceutical composition to bind to a specific nucleic acid sequence in a targeted manner, thereby treating or suppressing triple-negative breast cancer not only with a reduced dose of doxorubicin, but also with effectively reduced side effects of those chemotherapy drugs.

## Description

### BACKGROUND OF THE INVENTION

### 1. Technical Field

The present invention relates to a pharmaceutical composition and more particularly to a pharmaceutical composition for treating triple-negative breast cancer.

### 2. Description of Related Art

As pointed out by the World Health Organization, breast cancer is the most common cancer in females, with triple-negative breast cancer (TNBC) having the highest mortality rate. Triple-negative breast cancer refers to a breast cancer that tests negative for all the following three hormone receptors: the estrogen receptor (ER), the progesterone receptor (PR), and the human epidermal growth factor receptor 2 (HER2). Due to the lack, or low levels, of those receptors, a patient with such a cancer can use only a limited number of therapeutic methods. Moreover, triple-negative breast cancer has a high degree of genetic variation and therefore has a high chance of recurrence, or a poor prognosis, even after treatment. These explain why it remains a major clinical challenge to treat triple-negative breast cancer.

As a patient with triple-negative breast cancer lacks the aforesaid three receptors, hormone therapy and targeted therapy cannot be used for treatment, leaving only chemotherapy as the main therapeutic method. However, chemotherapy drugs have not only side effects such as cardiotoxicity and decreased immunity, but also an upper limit of cumulative dose. Once the maximum cumulative dose of a drug is reached, continued use of the drug is not advised; as a result, the patient's treatment alternatives are further reduced. For instance, doxorubicin, which is clinically the most common drug for chemotherapy of triple-negative breast cancer, has a maximum lifetime cumulative dose of 550 mg/m² (body surface area), and the guidelines for treating triple-negative breast cancer with doxorubicin are as follows: for monotherapy, the recommended dosage is 60-75 mg/m² once every three weeks; and for therapy in combination with other chemotherapy drugs, the recommended dosage is 40-75 mg/m² once every three to four weeks. A patient, therefore, can receive only a limited number of treatments with doxorubicin over the course of their life. Aside from chemotherapy, an emerging therapeutic method for triple-negative breast cancer is immunotherapy, but immunotherapy is not suitable for all patients with triple-negative breast cancer and is too expensive to be affordable to all patients.

### BRIEF SUMMARY OF THE INVENTION

To solve the aforesaid problems, the primary objective of the present invention is to provide a pharmaceutical composition for treating triple-negative breast cancer; for reducing the doses, and hence the side effects, of some known chemotherapy drugs to be taken by patients with triple-negative breast cancer; and for improving the patients' prognoses.

Another objective of the present invention is to provide a pharmaceutical composition for treating triple-negative breast cancer, for delaying the onset of diseases caused by the accumulation of chemotherapy drugs in a patient's body, and for thereby extending the time for which the patient can use the chemotherapy drugs.

To achieve the above objectives, the present invention provides a use of chemotherapy drugs in preparing a pharmaceutical composition for treating triple-negative breast cancer or metastasis thereof, wherein the chemotherapy drugs include actinomycin D and doxorubicin. By administering a pharmaceutical composition containing an effective amount of actinomycin D and doxorubicin to a patient with triple-negative breast cancer, the actinomycin D and the doxorubicin can be intercalated into the sequence GCCG, and this allows the pharmaceutical composition to bind to a nucleic acid molecule containing the sequence GCCG to inhibit the growth and metastasis of triple-negative breast cancer cells, thereby producing the effect of treating triple-negative breast cancer.

The pharmaceutical composition for treating triple-negative breast cancer is prepared from and composed of actinomycin D and doxorubicin.

In one embodiment of the present invention, the effective amount of the actinomycin D and the doxorubicin in the pharmaceutical composition refers to the dose ratio of the actinomycin D to the doxorubicin being in the range from 1:100 to 1:600.

In another embodiment of the present invention, if the pharmaceutical composition is administered to humans, the effective amount of the actinomycin D and the doxorubicin in the pharmaceutical composition refers to the dose ratio of the actinomycin D to the doxorubicin preferably being in the range from 1:10 to 1:30. For example, the dose ratio of the actinomycin D to the doxorubicin used is 1:25.

Another embodiment of the present invention discloses a use of chemotherapy drugs in preparing a nucleic acid intercalating agent (also referred to as a nucleic acid intercalator), wherein the chemotherapy drugs include actinomycin D and doxorubicin, and wherein the nucleic acid intercalating agent is used to bind to the sequence GCCG in a nucleic acid molecule. More specifically, by administering an effective amount of the nucleic acid intercalating agent to an individual, the actinomycin D and the doxorubicin can be intercalated into the sequence GCCG such that the nucleic acid intercalating agent and the nucleic acid molecule containing the sequence GCCG form a complex, which in turn affects the normal functions of the nucleic acid molecule containing the sequence GCCG or of the cell containing the nucleic acid molecule.

The dose ratio of the actinomycin D to the doxorubicin is in the range from 1:100 to 1:600.

If the nucleic acid intercalating agent is administered to humans, the dose ratio of the actinomycin D to the doxorubicin is preferably in the range from 1:10 to 1:30. For example, the dose ratio of the actinomycin D to the doxorubicin is 1:25.

The present invention has the following advantageous effects:

The pharmaceutical composition provided by the present invention for treating triple-negative breast cancer can reduce the doses, and hence the side effects, of some known chemotherapy drugs to be taken by patients with triple-negative breast cancer and can improve the patients' prognoses. The pharmaceutical composition can also delay the onset of diseases caused by the accumulation of chemotherapy drugs in a patient's body, thereby extending the time for which the patient can use the chemotherapy drugs. Furthermore, apart from suppressing the growth of triple-negative breast cancer cells, the pharmaceutical composition can effectively inhibit metastasis of triple-negative breast cancer to other organs; that is to say, the pharmaceutical composition provided by the invention can be used to treat metastatic triple-negative breast cancer or advanced triple-negative breast cancer.

### BRIEF DESCRIPTION OF THE SEVERAL VIEWS OF THE DRAWINGS

FIG. 1 schematically shows how the pharmaceutical composition disclosed herein for treating triple-negative breast cancer binds to the sequence d(AGCCGT)₂ to form a complex, wherein drug A is actinomycin D, and drug X is doxorubicin.
FIG. 2A shows the results of treating MDA-MB-231 cells with actinomycin D and doxorubicin in different ratios, wherein A represents actinomycin D, and X represents doxorubicin.
FIG. 2B shows the results of treating 4T1 cells with actinomycin D and doxorubicin in different ratios, wherein A represents actinomycin D, and X represents doxorubicin.
FIG. 2C shows the results of treating MCF-7 cells with actinomycin D and doxorubicin in different ratios, wherein A represents actinomycin D, and X represents doxorubicin.
FIG. 3 shows the calculation and analysis results of CI values obtained by assaying the cytotoxicity of actinomycin D and doxorubicin, in different ratios, in treating different cancer cells.
FIG. 4A shows the calculation results of summary synergy scores obtained by treating MDA-MB-231 cells with actinomycin D and doxorubicin in different ratios, wherein A represents actinomycin D, and DOX represents doxorubicin.
FIG. 4B shows the calculation results of summary synergy scores obtained by treating 4T1 cells with actinomycin D and doxorubicin in different ratios, wherein A represents actinomycin D, and DOX represents doxorubicin.
FIG. 4C shows the calculation results of summary synergy scores obtained by treating MCF-7 cells with actinomycin D and doxorubicin in different ratios, wherein A represents actinomycin D, and DOX represents doxorubicin.
FIG. 5 shows the cell cycle distributions of MDA-MB-231 cells and 4T1 cells treated with different treatment regimes.
FIG. 6A shows the statistical analysis results of the cell cycle distributions of MDA-MB-231 cells treated with different treatment regimes.
FIG. 6B shows the statistical analysis results of the cell cycle distributions of 4T1 cells treated with different treatment regimes.
FIG. 7 is a heatmap of the transcriptomes of MDA-MB-231 cells treated with different treatment regimes.
FIG. 8A shows the protein interaction network created for a combination therapy with respect to the control group.
FIG. 8B shows the protein interaction network created for a combination therapy with respect to the doxorubicin-treated group.
FIG. 9 shows the results of a thermal stability test performed on combinations of the sequence represented by SEQ ID NO. 1 and different drugs.
FIG. 10 shows the changes in melting temperature of differently treated groups.
FIG. 11 shows CD spectra obtained by reacting actinomycin D, doxorubicin, and a combination of actinomycin D and doxorubicin with different sequences, wherein A represents actinomycin D, and X represents doxorubicin.
FIG. 12 shows the changes in tumor volume of different groups of mice.
FIG. 13 shows the changes in leukocyte content of different groups of mice.
FIG. 14 shows the changes in body weight of different groups of mice.
FIG. 15 shows an analysis result of the tumor weights of different groups of mice.
FIG. 16 shows an analysis result of the liver weight of each of different groups of mice with respect to the corresponding body weight.
FIG. 17 shows an analysis result of the heart weight of each of different groups of mice with respect to the corresponding body weight.
FIG. 18 shows an analysis result of the numbers of lung metastatic nodules in different groups of mice.
FIG. 19 shows an analysis result of the numbers of liver metastatic nodules in different groups of mice.
FIG. 20 shows H&E stained lung and liver sections of differently treated mice.

### DETAILED DESCRIPTION OF THE INVENTION

The present invention provides a pharmaceutical composition for treating triple-negative breast cancer, and the pharmaceutical composition includes actinomycin D and doxorubicin. The actinomycin D and the doxorubicin can intercalate into the sequence GCCG after an effective amount of the pharmaceutical composition provided by the invention for treating triple-negative breast cancer is administered to a patient with breast cancer, and this allows the pharmaceutical composition to bind, in a targeted manner, to a nucleic acid molecule containing the sequence GCCG in order to treat, or inhibit the growth and metastasis of, triple-negative breast cancer and reduce the side effects of drugs.

When actinomycin D and doxorubicin are combined in a ratio within a predetermined range, the two drugs can produce a synergistic effect by which the actinomycin D can, together with the doxorubicin, be intercalated into the GCCG sites of a nucleic acid such that the two drugs and the nucleic acid sequence form a complex. In addition, the actinomycin D enables the doxorubicin to bind to the nucleic acid sequence GCCG in a targeted manner, thereby reducing the possibility that the doxorubicin may bind to non-target sequences. This effectively reduces the dose to be used of the doxorubicin so that not only is the rate of occurrence of the side effects of the drug reduced, but also the time for which the drug can be administered to a the patient is extended.

As far as breast cancer cells, in particular triple-negative breast cancer cells, are concerned, the pharmaceutical composition disclosed herein can inhibit the growth of such cells when the dose administered of the actinomycin D and the dose administered of the doxorubicin (or the concentration at which the actinomycin D is administered and the concentration at which the doxorubicin is administered) are in a ratio of 1:100 to 1:600, such as 1:100, 1:200, 1:300, 1:400, 1:500, or 1:600. More specifically, when the dose of the actinomycin D is 20 µg/kg, the dose of the doxorubicin may be 2000-12000 µg/kg; when the concentration of the actinomycin D is 2 nM, the concentration of the doxorubicin may be 200-1200 nM.

In one embodiment of the present invention, with the pharmaceutical composition administered to a human, the aforesaid effective amount refers to the dose ratio of the actinomycin D to the doxorubicin preferably being in the range from 1:10 to 1:30, such as 1:15, 1:20, or 1:22, more preferably 1:25.

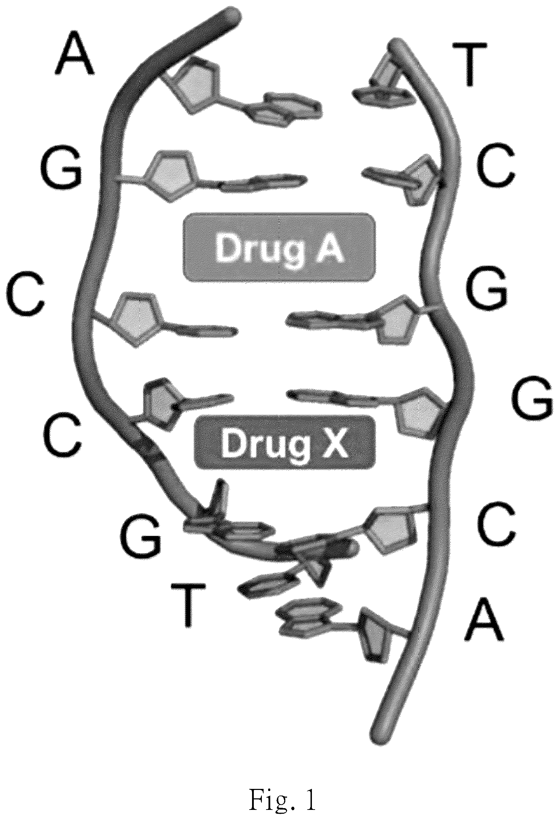

As shown in FIG. 1, the pharmaceutical composition disclosed herein for treating triple-negative breast cancer is such that the actinomycin D and the doxorubicin contained in the pharmaceutical composition can bind to the sequence d(AGCCGT)₂ to form a complex, hindering the growth and metastasis of cancer cells, thereby treating or improving triple-negative breast cancer.

The pharmaceutical composition disclosed herein for treating triple-negative breast cancer uses the synergistic effect between actinomycin D and doxorubicin to inhibit RAS pathway-related genes. Therefore, the doses of actinomycin D and doxorubicin can be reduced by more than one half while still being able to inhibit triple-negative breast cancer, and this allows the side effects of the drugs to be lessened.

As actinomycin D and doxorubicin can be intercalated into the sequence GCCG of a nucleic acid molecule in a targeted manner, another embodiment of the present invention discloses a nucleic acid intercalating agent whose active ingredients include actinomycin D and doxorubicin and which can be used to bind to the sequence GCCG of a nucleic acid molecule.

The term "triple-negative breast cancer" refers to the breast cancer subtype in which the estrogen receptor (ER), the progesterone receptor (PR), and the human epidermal growth factor receptor 2 (HER2) are not expressed. This subtype constitutes about 15% of all breast cancer cases. In the present invention, "triple-negative breast cancer" includes metastatic triple-negative breast cancer, late-stage triple-negative breast cancer, and early-stage triple-negative breast cancer.

The term "pharmaceutical composition" refers to a composition that includes at least two active ingredients for treating a disease or the related symptoms, with the active ingredients having a synergistic effect between them.

The term "treating" refers to reversing, alleviating, or inhibiting the progression, or one or more symptoms, of the disease to which the term is applied or preventing the occurrence of the disease to which the term is applied or of complications thereof.

The term "effective amount" refers to an amount in which a compound or a pharmaceutically acceptable salt thereof is administered and that can alleviate one or more symptoms of the disease to be treated, reduce the discomfort of one or more symptoms of the disease, or slow down the progression of the disease.

The term "actinomycin D (abbreviated as ActD)," also referred to as dactinomycin, is an actinomycin-based polypeptide antibiotic that is separated from *Streptomyces* bacteria in soil and usable as a chemotherapy drug. Actinomycin D can inhibit RNA synthesis and stop protein synthesis and is used mainly to treat Wilm's tumor, testicular cancer, and rhabdomyosarcoma.

The term "doxorubicin (abbreviated as Dox)" is a compound having the chemical formula (I) shown below, a molecular mass of 543.52 g/mol, and the molecular formula C₂₇H₂₉NO₁₁. Doxorubicin is a drug for treating a variety of cancers, is an anthracycline antibiotic, and works mainly by binding to nuclear DNA to inhibit DNA and RNA synthesis and thereby interfere with the multiplication and fission of cancer cells. In clinical treatment of breast cancer, doxorubicin is usually part of a chemotherapy regime. For example, doxorubicin can be used in combination with cyclophosphamide to treat invasive breast cancer. When used together with other chemotherapy drugs, however, doxorubicin has such side effects as bone marrow suppression (damage to haematopoiesis), nausea, vomiting, hair loss, cardiotoxicity (damage to cardiac muscles), and heptatoxicity. The most serious side effect of doxorubicin is dilated cardiomyopathy (DCM). Not only is the incidence rate of DCM positively correlated to the cumulative dose of doxorubicin, but also there is no clinically effective method for treating DCM. This is why a patient with triple-negative breast cancer cannot use doxorubicin as a long-term means of treatment.

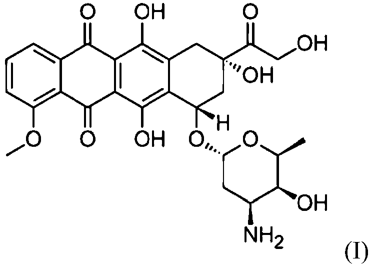

The cell lines used in the following examples, namely the MDA-MB-231 (human triple-negative breast cancer) cell line, the 4T1 (mouse triple-negative breast cancer) cell line, and the MCF-7 (breast cancer) cell line, are easily obtainable by a person skilled in the art and therefore need not be deposited. The 4T1 cell line is frequently used as a research model for the metastasis of breast cancer and a model for the selection of clinical drugs for breast cancer.

The sequences used in the following examples, such as d(AGCCCT)₂ and d(AGCACGT)₂, are artificial synthetic sequences and were used not to limit the effect of the present invention but to verify that the pharmaceutical composition disclosed herein can target the sequence GCCG.

The doses used in the cell tests and the animal test in the following examples are provided for illustrative purposes only and are not intended to be restrictive of the scope of the present invention.

### Example 1: Test on synergistic cytotoxic effect

Table 1 below shows the half maximal inhibitory concentrations (IC₅₀) of various chemotherapy drugs, namely actinomycin D, doxorubicin, cisplatin, and 5FU, against MDA-MB-231 cells, 4T1 cells, and MCF-7 cells.

**Table 1: IC₅₀ of various chemotherapy drugs against different cancer cells**

| Cell line | Actinomycin D | Doxorubicin | Cisplatin | 5FU |
|---|---|---|---|---|
| MDA-MB-231 cells | 1.1±0.19 µM | 6.5±0.9 nM | 37.2±5.1 µM | >100 µM |
| 4T1 cells | 0.5±0.04 µM | 2.8±1.1 nM | 3.3±1.2 µM | 5.9±0.3 µM |
| MCF-7 cells | 0.3±0.06 µM | 4.5±1.7 nM | 19.1±2.2 µM | 38.3±5.0 µM |

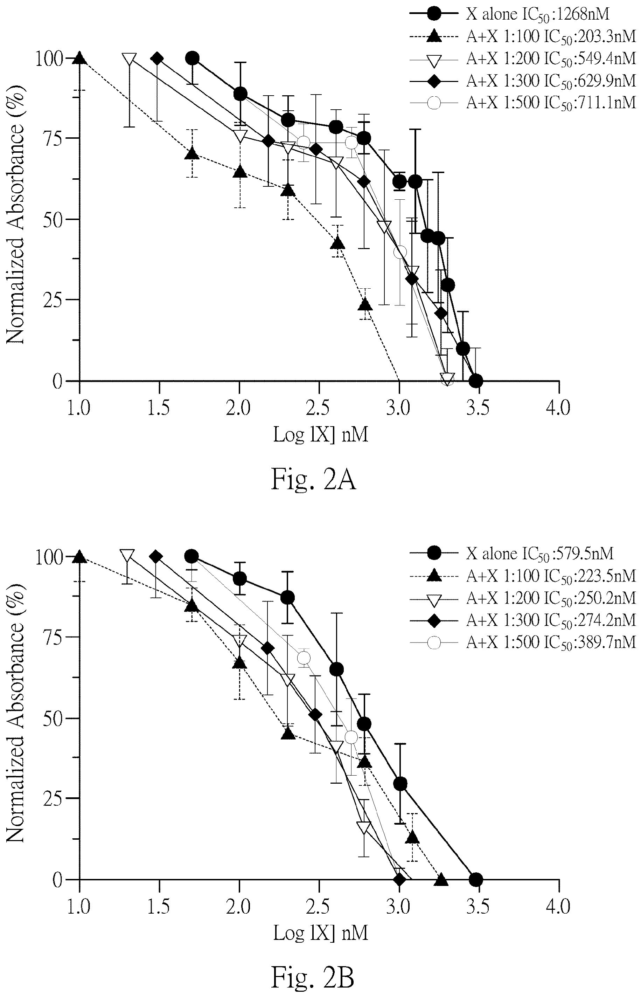

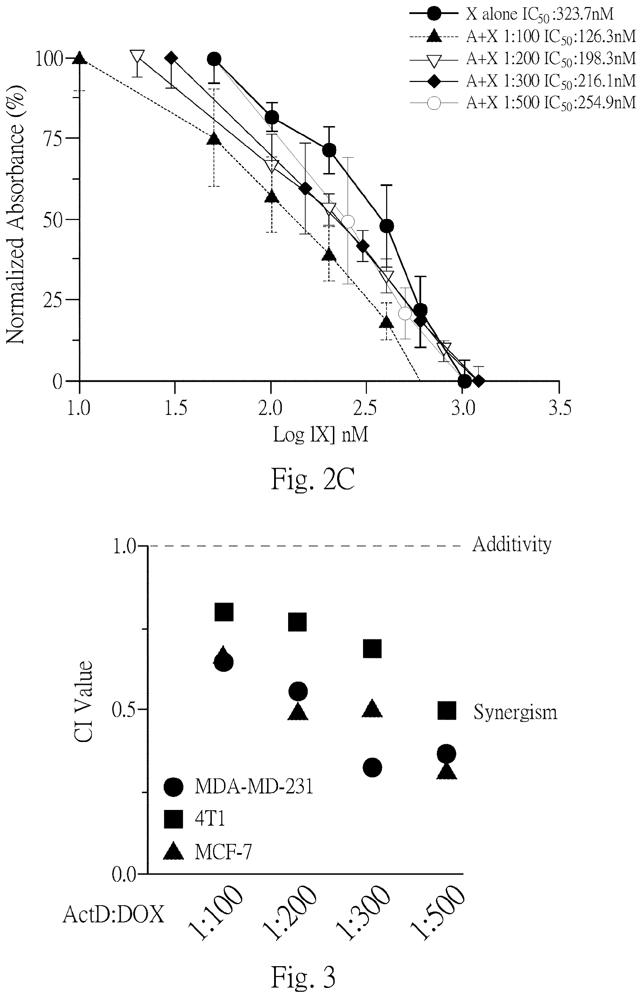

MDA-MB-231 cells, 4T1 cells, and MCF-7 cells were treated with actinomycin D and doxorubicin in different ratios for 48 hours, before combination indices (CI) were calculated based on cytotoxicity. The results are shown in FIG. 2, FIG. 3, and Table 2, with a CI value less than 0.9 indicating that the interaction between the drug components of the corresponding combination was synergistic, and a CI value between 0.9 and 1.0 indicating that the interaction between the drug components was additive. It can be known from the results in FIG. 3 that the CI values of all the combinations of actinomycin D and doxorubicin in different ratios are less than 0.9, and this demonstrates the synergistic effect between actinomycin D and doxorubicin.

**Table 2: CI values corresponding to differently treated cell groups**

| Cell line | IC₅₀ | | CI (ED₅₀) | | | |
|---|---|---|---|---|---|---|
| | | | Actinomycin D:Doxorubicin | | | |
| | Actinomycin | Doxorubicin | 1:100 | 1:200 | 1:300 | 1:600 |
| MDA-MB-231 cells | 3.3 nM | 1.268 µM | 0.65 | 0.56 | 0.33 | 0.37 |
| 4T1 cells | 2.6 nM | 0.579 µM | 0.8 | 0.77 | 0.69 | 0.50 |
| MCF-7 cells | 3.7 nM | 0.323 µM | 0.67 | 0.5 | 0.51 | 0.32 |

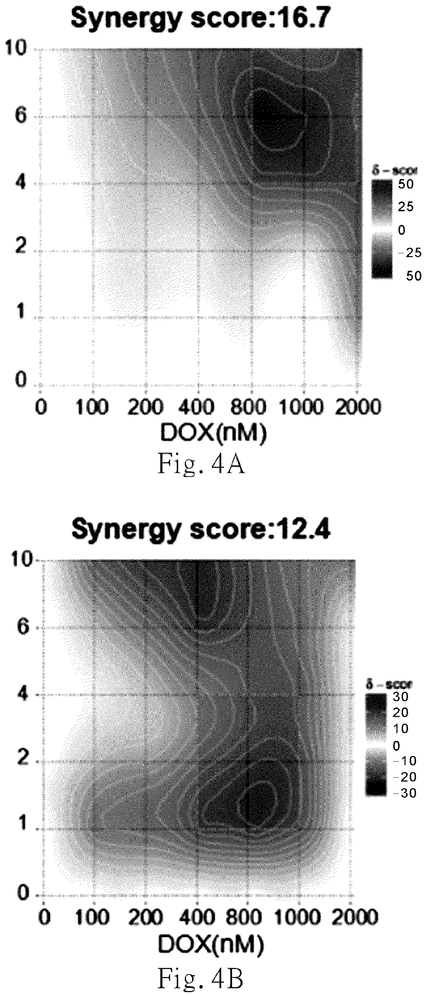

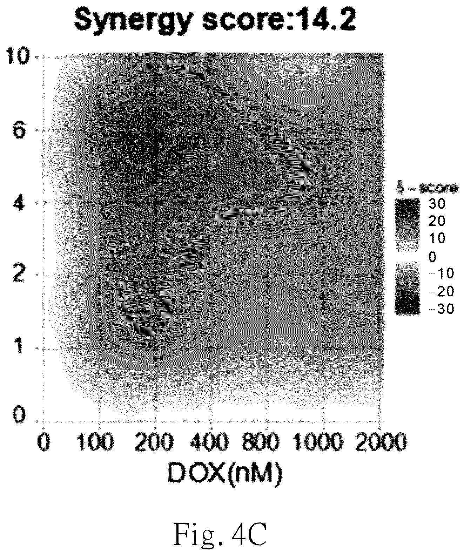

In addition, a cross-analysis and visualization of the reaction data of actinomycin D and doxorubicin were performed by computing with SynergyFinder, and the results are shown in FIG. 4, from which it can be known that all the summary synergy scores are greater than 10, meaning that actinomycin D and doxorubicin interacted with each other synergistically against various breast cancer cells.

### Example 2: Cell test (1)

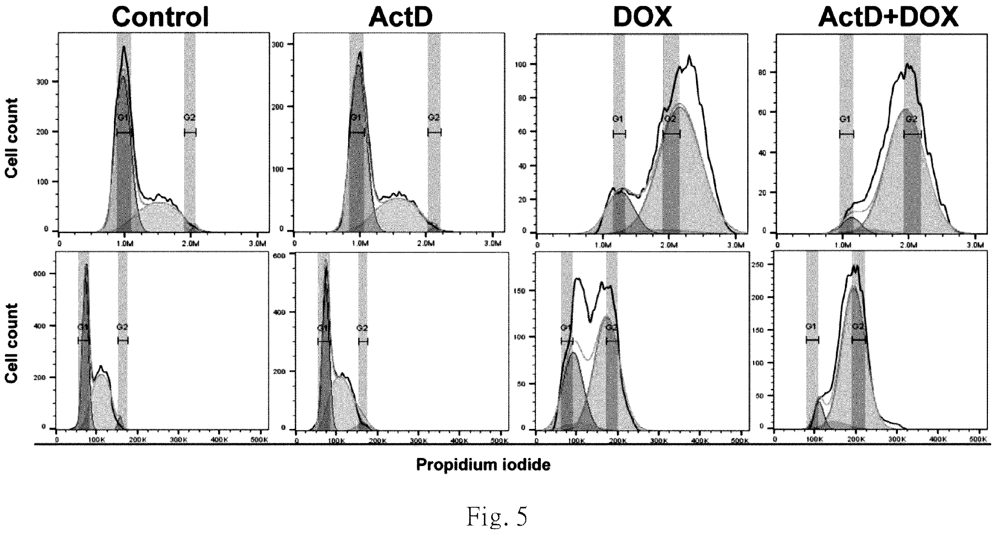

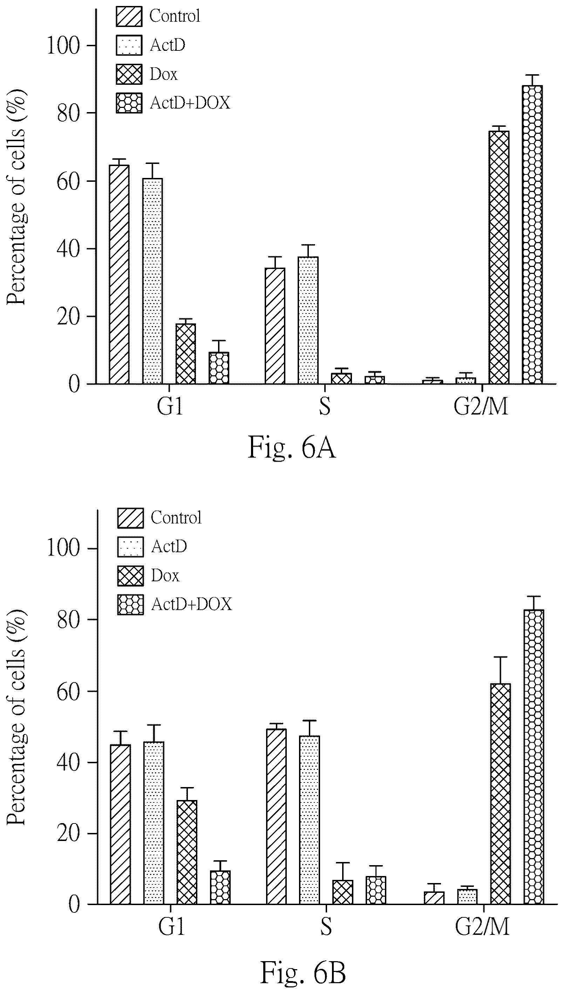

MDA-MB-231 cells and 4T1 cells were treated with actinomycin D (0.25 nM), doxorubicin (5 nM), or a combination of actinomycin D (0.25 nM) and doxorubicin (5 nM) for 24 hours. After that, nucleic acids were marked by staining with propidium iodide (PI), and a cell cycle analysis was carried out using a flow cytometer. The results are shown in FIG. 5 and FIG. 6.

It can be inferred from the results in FIG. 5 and FIG. 6 that actinomycin D can promote the arrest of the cell cycle of triple-negative breast cancer cells in the G2/M phase by doxorubicin. That is to say, the pharmaceutical composition disclosed herein for treating triple-negative breast cancer can effectively inhibit the growth of, and thereby treat, triple-negative breast cancer.

### Example 3: Cell test (2)

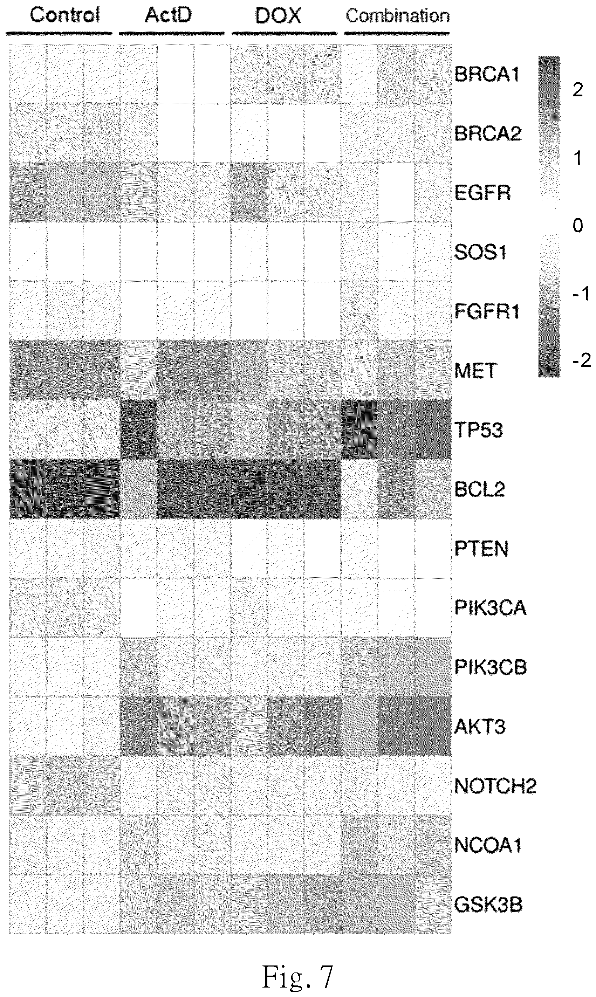

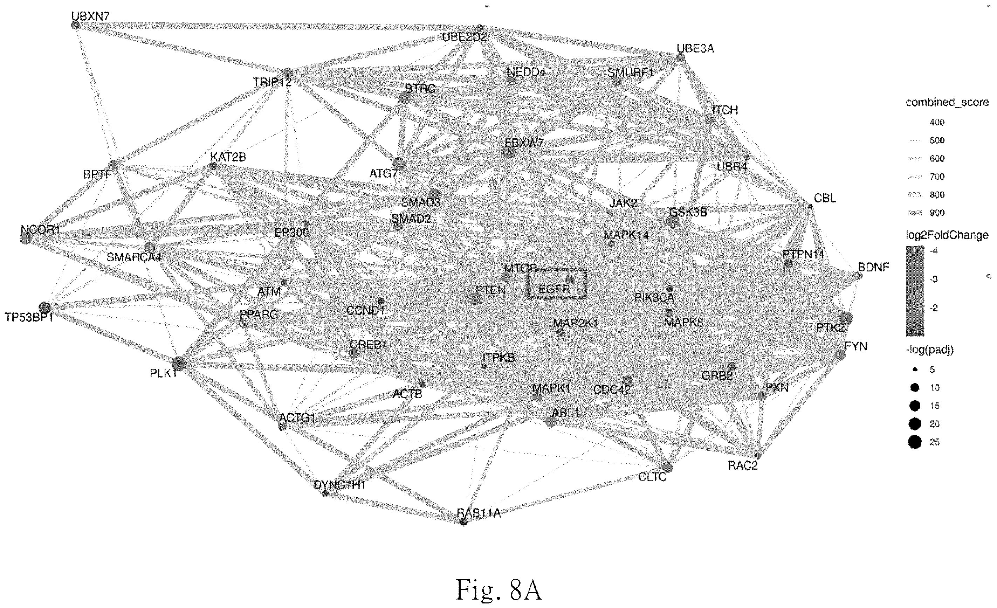

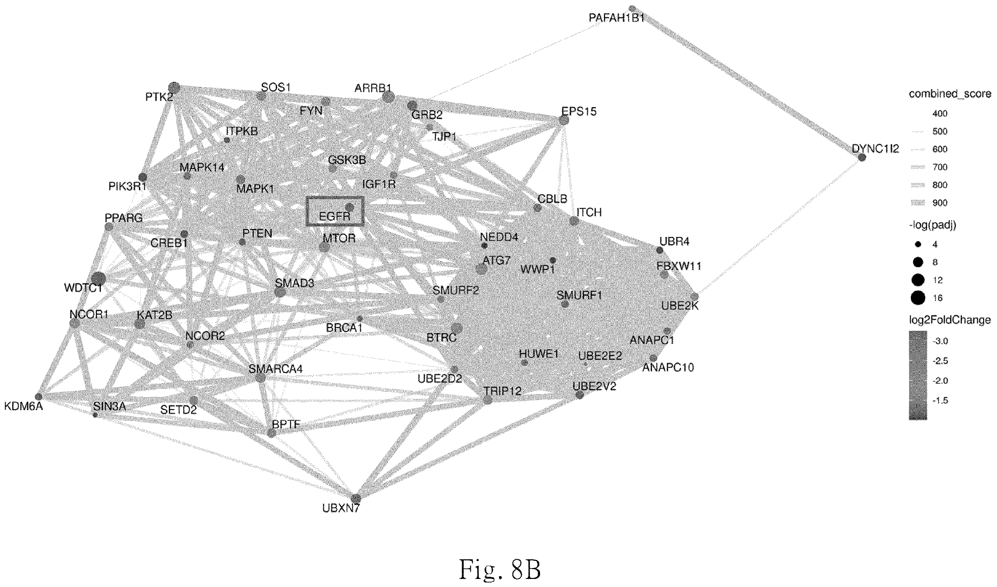

MDA-MB-231 cells were treated with actinomycin D (2 nM), doxorubicin (600 nM), or a combination of actinomycin D (2 nM) and doxorubicin (600 nM) for 24 hours, during which time changes in the transcriptome of each cell group were recorded, as shown in FIG. 7. Furthermore, the protein interaction network of each cell group was analyzed with software, and the results are shown in FIG. 8.

### Example 4: Test on specificity of binding to sequence GCCG

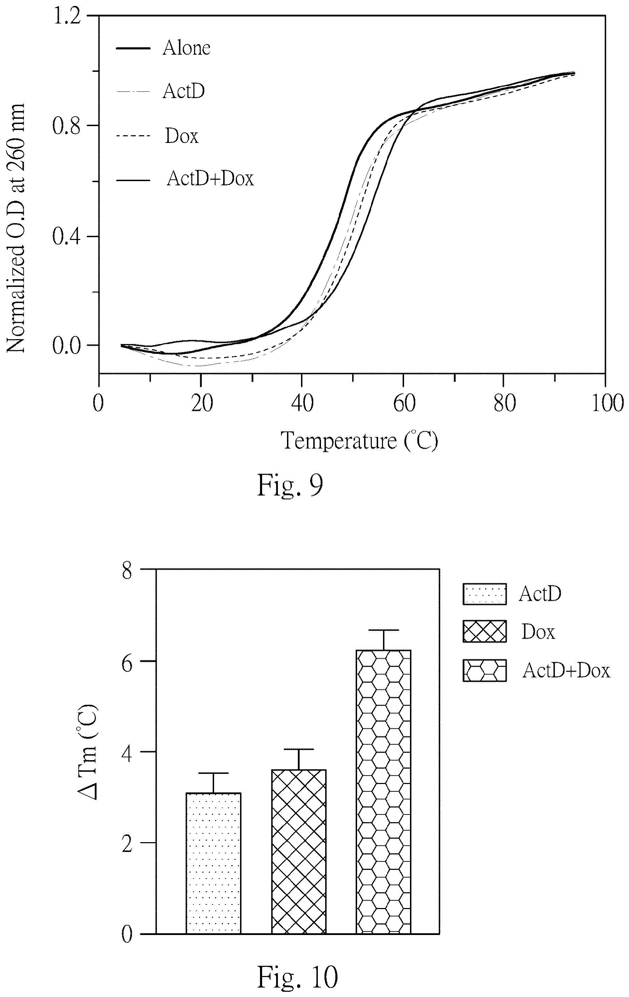

The sequence represented by SEQ ID NO. 1 (at a concentration of 2 µM) was reacted with actinomycin D, doxorubicin, or a combination of actinomycin D and doxorubicin. In each group that was treated with a single drug, the dose ratio of the sequence to the single drug was 1:1, and in the group treated with the actinomycin D and the doxorubicin in the pharmaceutical composition disclosed herein, the dose ratio of the sequence to actinomycin D and doxorubicin was 1:1:1. The binding of the drug(s) to the sequence in each group and the changes in melting temperature of the sequence in each group were analyzed, and the results are shown in FIG. 9 and FIG. 10. It can be inferred from the results in FIG. 9 and FIG. 10 that actinomycin D and doxorubicin can form a complex with the sequence represented by SEQ ID NO. 1.

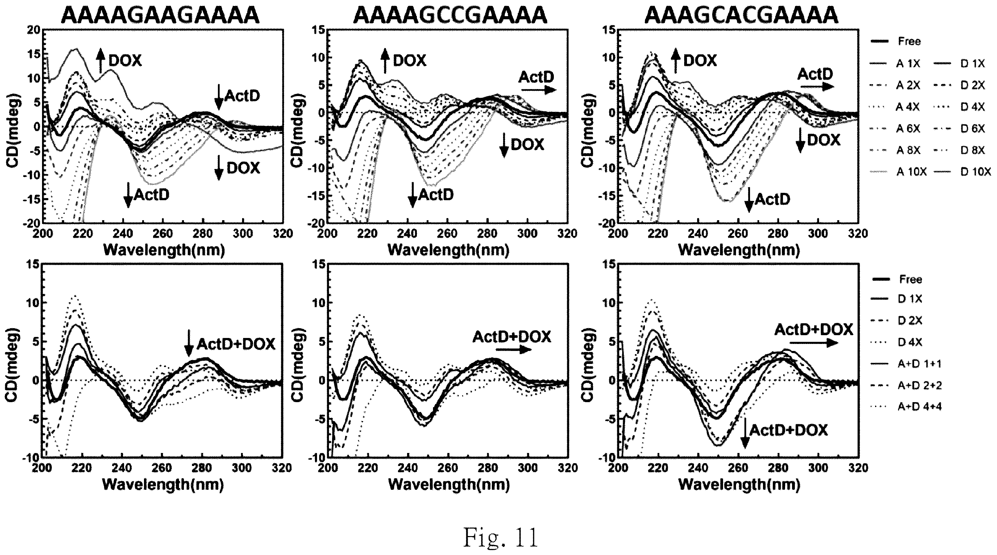

Moreover, the artificial synthetic sequences listed in Table 4 were each reacted with actinomycin D, doxorubicin, or a combination of actinomycin D and doxorubicin at different doses or in different ratios, and the circular dichroism (CD) spectrum of each of the differently treated groups was analyzed. The results are shown in FIG. 11. It can be inferred from the results in FIG. 11 that actinomycin D can bind to the nucleic acid sequence GCCG together with doxorubicin, meaning the pharmaceutical composition disclosed herein for treating triple-negative breast cancer can bind to the nucleic acid sequence GCCG in a targeted manner so that even though the dose of doxorubicin is reduced, the pharmaceutical composition can still treat triple-negative breast cancer.

**Table 4: List of artificial synthetic sequences**

| NO. | Sequence |
|---|---|
| SEQ ID NO.1 | AAAAGAAGAAAA |
| SEQ ID NO.2 | AAAAGCCGAAAA |
| SEQ ID NO.3 | AAAGCACGAAAA |

### Example 5: Animal test

MDA-MB-231 breast cancer mouse models were created based on common knowledge in the art. The mice in each of the following groups were treated on the 0^{th}, 7^{th}, 14^{th}, 21^{st}, 28^{th}, 35^{th}, 42^{nd}, and 49^{th} day of the test:
Group 1: Without any treatment;
Group 2: With actinomycin D administered at a dose of 20 µg/kg (equivalent to a clinical dose of 15 µg/kg for humans);
Group 3: With doxorubicin administered at a dose of 0.5 mg/kg (equivalent to a clinical dose of 20 mg/m² for humans);
Group 4: With both actinomycin D (20 µg/kg) and doxorubicin (0.5 mg/kg) administered; and
Group 5: With both actinomycin D (10 µg/kg) and doxorubicin (0.25 mg/kg) administered, the doses being 0.5 times as high as those for group 4.

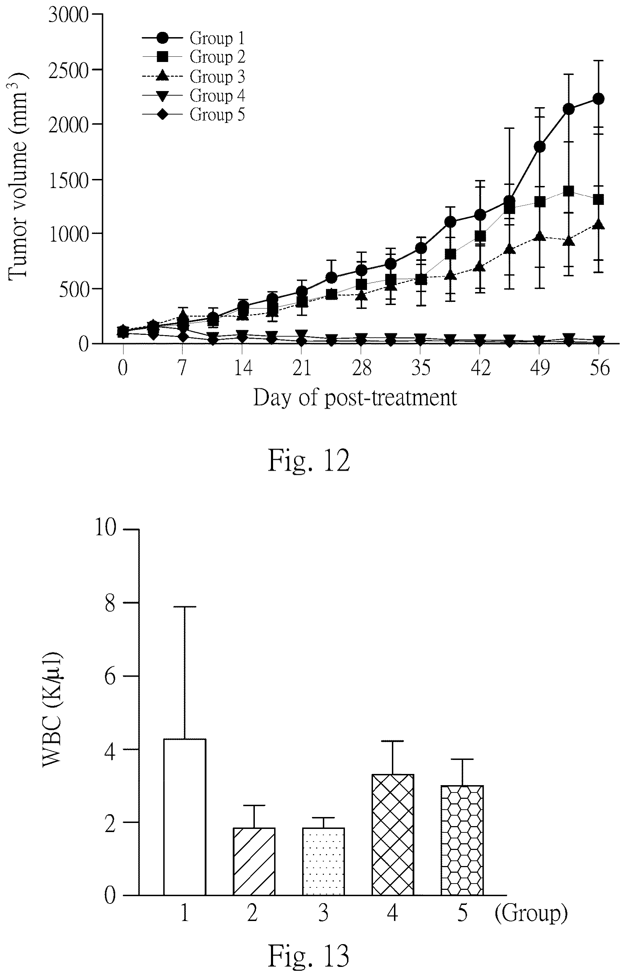

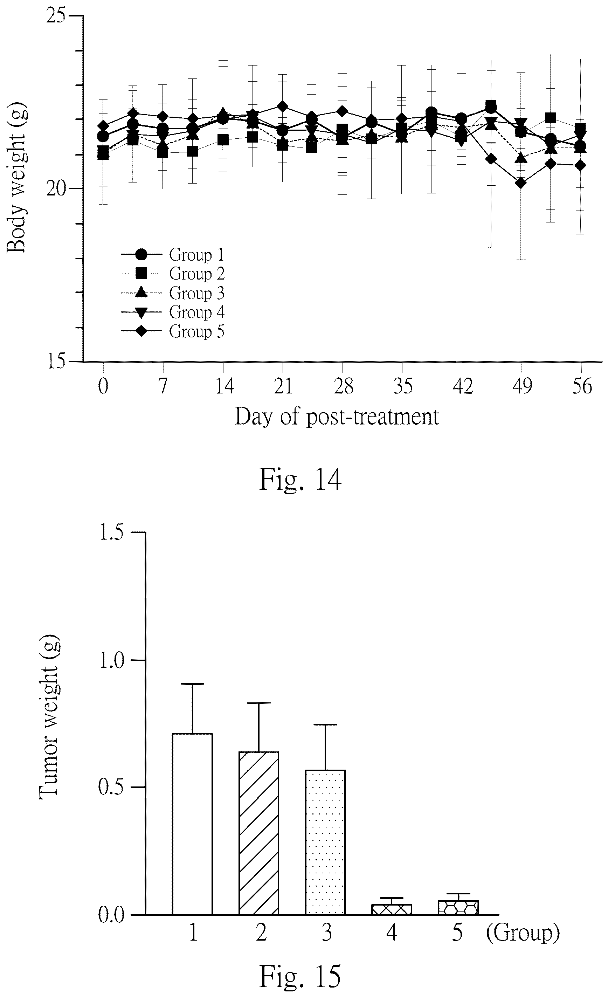

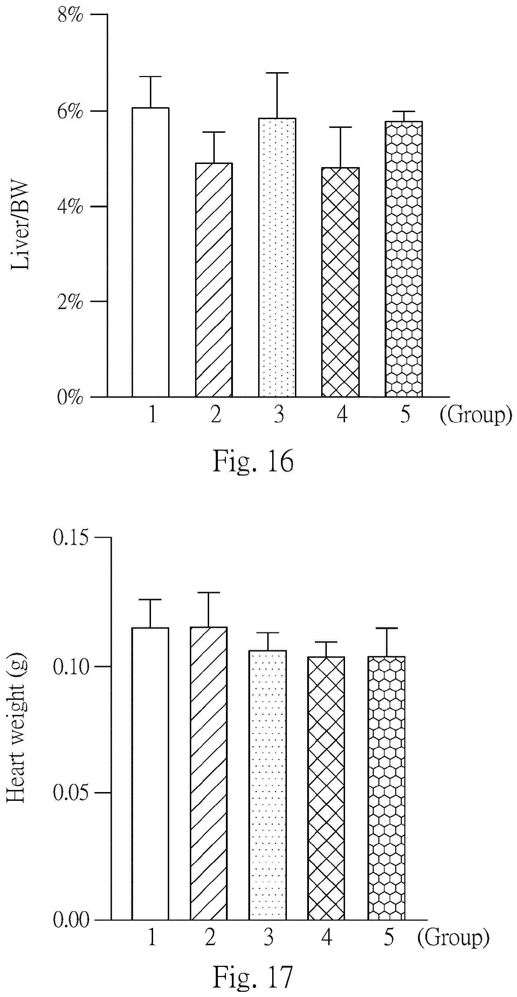

During the test, the changes in tumor volume, leukocyte content, and body weight of each group of mice were recorded, as shown in FIG. 12 to FIG. 14. After the test, the tumor weight, liver weight, and heart weight of each group of mice were analyzed, as shown in FIG. 15 to FIG. 17.

It can be inferred from the results in FIG. 12 to FIG. 17 that administering the pharmaceutical composition disclosed herein for treating triple-negative breast cancer to an individual with triple-negative breast cancer has no effect on the individual's body weight, liver weight, or heart weight. This indicates the safety of the disclosed pharmaceutical composition. Moreover, the pharmaceutical composition disclosed herein for treating triple-negative breast cancer can effectively reduce tumor volume and tumor weight when administered at 0.5 times as well as 1 times the predetermined dose, and this proves that the synergistic effect between the actinomycin D and the doxorubicin in the disclosed pharmaceutical composition makes it possible to reduce the administered dose required for actinomycin D and doxorubicin to be therapeutically effective.

It can be known from the above that the disclosed pharmaceutical composition allows the administered dose of actinomycin D and of doxorubicin to be reduced by at least half and can therefore effectively reduce the side effects of actinomycin D and doxorubicin on an individual, extend the time for which the drugs can be administered to the individual, and improve the therapeutic effect and prognosis.

### Example 6: Test on inhibition of cell migration

4T1 breast cancer mouse models were created based on common knowledge in the art. The test lasted for five weeks, and the mice in each of the following groups were treated on the 0^{th}, 7^{th}, 14^{th}, 21^{st}, 28^{th}, and 35^{th} day of the test:
Group 1: Without any treatment;
Group 2: With cyclophosphamide administered at a dose of 5 mg/kg;
Group 3: With both cyclophosphamide and doxorubicin administered, the dose of cyclophosphamide being 5 mg/kg, and the dose of doxorubicin being 0.5 mg/kg (equivalent to a clinical dose of 20 mg/m² for humans);
Group 4: With actinomycin D administered at a dose of 20 µg/kg (equivalent to a clinical dose of 15 µg/kg for humans);
Group 5: With doxorubicin administered at a dose of 0.5 mg/kg;
Group 6: With both actinomycin D (20 µg/kg) and doxorubicin (0.5 mg/kg) administered; and
Group 7: With both actinomycin D (10 µg/kg) and doxorubicin (0.25 mg/kg) administered, the doses being 0.5 times as high as those for group 4.

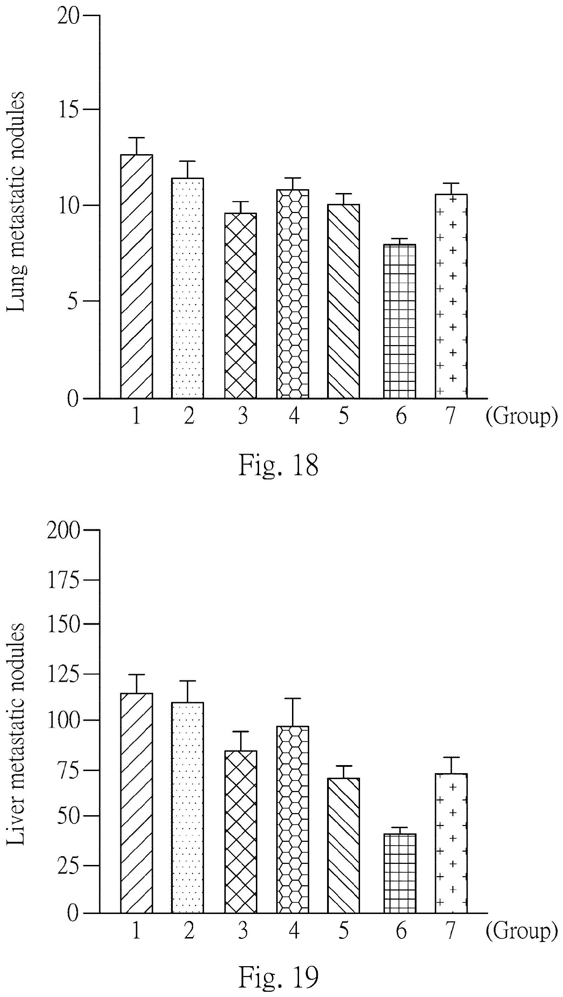

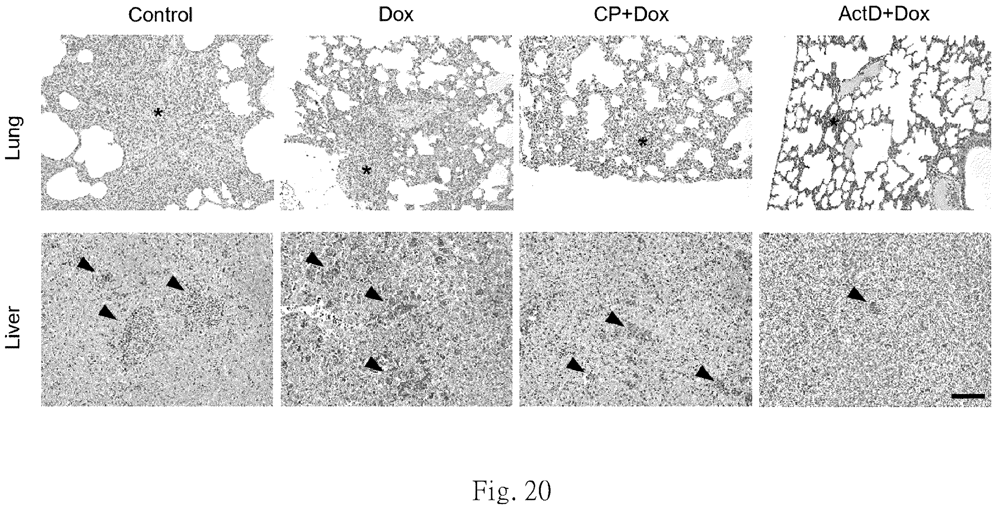

Once the test was completed, the mice in each group were sacrificed, and the numbers of breast tumors that had metastasized to the lungs and to the liver (i.e., lung metastatic nodules and liver metastatic nodules) of each group of mice were counted. The results are shown in FIG. 18 and FIG. 20. In addition, lungs and livers were collected from the mice, sectioned, and then stained with hematoxylin and eosin (H&E) stain, as shown in FIG. 20.

According to the results in FIG. 18 to FIG. 20, the number of liver metastatic nodules and the number of lung metastatic nodules in group-2 and group-3 mice were reduced only slightly in comparison with those of group-1 mice, and this indicates that administering cyclophosphamide alone or in combination with doxorubicin did not produce a significant therapeutic effect on the metastasis of breast cancer. The number of lung metastatic nodules and the number of liver metastatic nodules in group-4 mice were increased in comparison with those of group-3 mice, and this indicates that administering actinomycin D alone was not effective in inhibiting the metastasis of breast cancer. Group-5 mice had approximately the same number of lung metastatic nodules as group-3 mice and significantly less liver metastatic nodules than group-1 mice, and this indicates that administering doxorubicin alone was effective only in inhibiting metastasis to certain organs and, generally speaking, was unable to inhibit the metastasis of breast cancer effectively. Group-6 mice had significantly less liver metastatic nodules and lung metastatic nodules than the mice in the other groups, and this indicates that administering the disclosed pharmaceutical composition was effective in treating metastatic breast cancer. Group-7 mice were also given the disclosed pharmaceutical composition, but the administered doses of actinomycin D and doxorubicin were half of those given to group-6 mice and therefore not as effective in inhibiting the metastasis of breast cancer cells to the liver and the lungs as those given to group-6 mice.

The results of this example prove that the disclosed pharmaceutical composition not only can inhibit the growth of triple-negative breast cancer, but also is effective in inhibiting the metastasis of triple-negative breast cancer to other organs, meaning the disclosed pharmaceutical composition can be used to treat metastatic triple-negative breast cancer or late-stage triple-negative breast cancer.

## Claims

1. A use of chemotherapy drugs in preparing a pharmaceutical composition for treating triple-negative breast cancer or metastasis thereof, **characterized in that** the chemotherapy drugs comprise actinomycin D and doxorubicin.

2. The use of claim 1, wherein the pharmaceutical composition is composed of the actinomycin D and the doxorubicin.

3. The use of claim 1 or 2, wherein when the pharmaceutical composition is administered to humans, a dose ratio of the actinomycin D to the doxorubicin is in a range from 1:10 to 1:30.

4. The use of claim 3, wherein when the pharmaceutical composition is administered to humans, the dose ratio of the actinomycin D to the doxorubicin is 1:25.

5. The use of claim 1 or 2, wherein the pharmaceutical composition is used to bind to the nucleic acid sequence GCCG in a targeted manner.

6. A use of chemotherapy drugs in preparing a nucleic acid intercalating agent, **characterized in that** the chemotherapy drugs comprise actinomycin D and doxorubicin, and that the nucleic acid intercalating agent is used to bind to the sequence GCCG in a nucleic acid molecule.

7. The use of claim 6, wherein the nucleic acid intercalating agent is composed of the actinomycin D and the doxorubicin.

8. The use of claim 6 or 7, wherein when the nucleic acid intercalating agent is administered to humans, a dose ratio of the actinomycin D to the doxorubicin is in a range from 1:10 to 1:30.

9. The use of claim 8, wherein the dose ratio of the actinomycin D to the doxorubicin is 1:25.
